# EUROPÄISCHE PATENTSCHRIFT

(11) **EP 2 814 800 B1**
(45) Veröffentlichungstag und Bekanntmachung des Hinweises auf die Patenterteilung: **13.09.2017**
(21) Anmeldenummer: 13706187.5
(22) Anmeldetag: 07.02.2013
(51) Int. Cl.: C07C 29/34, C07C 31/125, A61K 8/34, A61Q 19/00, A61K 9/00

(54) **GUERBETALKOHOLE ALS VASELINE-ERSATZ**
GUERBET ALCOHOLS AS VASELINE SUBSTITUTE
ALCOOL DE GUEBERT EN TANT QUE REMPLACEMENT DE LA VASELINE

(30) Priorität: 17.02.2012 US 201261599965 P; 17.02.2012 EP 12155971
(43) Veröffentlichungstag der Anmeldung: 24.12.2014
(73) Patentinhaber: BASF SE, 67056 Ludwigshafen am Rhein (DE)
(72) Erfinder: KEMPERS, Peter, 41189 Mönchengladbach (DE); KAWA, Rolf, 40789 Monheim (DE); BRÜNING, Stefan, 40593 Düsseldorf (DE); MILARDOVIC, Jadranka, 40591 Düsseldorf (DE); MAHNKE, Eike, Ulf, 42553 Velbert (DE); KRÜPPEL, Heinz-Josef, 41515 Grevenbroich (DE); DIERKER, Markus, 40593 Düsseldorf (DE); BECKEDAHL, Burkhard, 40589 Düsseldorf (DE)
(74) Vertreter: BASF IP Association
(86) Internationale Anmeldenummer: PCT/EP2013/052417
(87) Internationale Veröffentlichungsnummer: WO 2013/120757

(56) Entgegenhaltungen:
- EP-A1- 0 970 998
- WO-A1-2004/006869
- US-A- 3 553 192
- US-A1- 2011 150 805
- Goetz Lachmann: "Sasol Olefins & Surfactants ISOFOL C12-C32 Defined Branched Guerbet Alcohols", , 1. Oktober 2003 (2003-10-01), Seiten 1-12, XP055031579, Gefunden im Internet: URL:http://www.sasoltechdata.com/tds/ISOFO L.pdf [gefunden am 2012-07-03]
- M. Sulzbacher: "THE GUERBET REACTION OF CETYL ALCOHOL", J. Appl. Chem., 1. Januar 1955 (1955-01-01), Seiten 637-641, XP055031557, DOI: 10.1002/jctb.5010051201 Gefunden im Internet: URL:http://onlinelibrary.wiley.com/doi/10. 1002/jctb.5010051201/abstract [gefunden am 2012-07-03]

## Beschreibung

### Gebiet der Erfindung

Die vorliegende Erfindung betrifft Gemische von Guerbetalkoholen, ihre Verwendung in kosmetischen und/oder pharmazeutischen Zubereitungen als Vaselineersatz, sowie kosmetische und/oder pharmazeutische Zubereitungen, enthaltend Gemische von Guerbetalkoholen.

### Stand der Technik

Vaseline ist ein altbewährter Bestandteil vieler kosmetischer und/oder pharmazeutischer Grundlagen für die topische Anwendung. Sie findet eine sehr breite Anwendung in leave-on und rinse-off Zubereitungen, als Grundlage von Cremes und Salben und kann beispielsweise auch in Duschbädern eingesetzt werden.
Vaseline zählt zu den Kohlenwasserstoffgelen und stellt ein Zweiphasensystem mit 70 bis 90 % einer flüssigen Phase aus n- und Isoparaffinen und Olefinkohlenwasserstoffen wie Ceten, Heptadecen und Octadecen, sowie 10 bis 30 % einer festen Phase. Die feste Phase besteht aus einem mikrokristallinen Anteil überwiegend von Isoparaffinen und geringen Anteilen Alicyclen und einem kristallinen Anteil aus n-Paraffinen. Die Gelstruktur der Vaseline entsteht durch die Ausbildung eines Gerüstes, das durch die längerkettigen festen Paraffine gebildet wird. Diese lagern sich - über van der Waals-London Kräfte gehalten - parallel aneinander und bilden sogenannte Fransenmicele, da die Enden der langketttigen Paraffine ungleichmäßig aus dem Micel herausragen und teilweise zur Bildung weiterer Micele beitragen. So entsteht ein dreidimensionales Gerüst durch zahlreiche miteinander verknüpfte Inseln parallel gelagerter langkettiger Paraffine, in das die flüssigen Kohlenwasserstoffe eingelagert werden. Die entsprechende Zusammensetzung von kristallinen, mikrokristallinen Bereichen und flüssigen Kohlenwasserstoffen bestimmt die Bildung dieser Gelstruktur und damit die besonderen rheologischen Eigenschaften (Plastizität, Duktilität) der Vaseline.

Vaseline zeichnet sich durch einen sehr breiten Schmelzbereich von ca. -10 bis +60°C aus und verhält sich chemisch weitgehend neutral.

Überwiegend wird natürlich gewonnene Vaseline in kosmetischen und pharmazeutischen Zubereitungen verwendet, die ein bei der Erdölraffination im Rückstand anfallendes Gemisch von n-Paraffinen, Isoparaffinen und hydroaromatischen Kohlenwasserstoffen darstellt, das durch Behandlung mit konzentrierter Schwefelsäure und Bleicherden und/oder Aktivkohle aufgereinigt wird. Je nach Art der Aufreinigung entstehen unterschiedliche Qualitäten der Vaseline. Ebenso existiert aber auch eine synthetisch hergestellte Vaseline, die durch Auflösen von Paraffin und Ceresin in flüssigem Paraffin gewonnen wird.

Von Paraffinen ist jedoch bekannt, dass sie sich je nach Kettenlänge in Leber, Lymphknoten und Niere anreichern können. Immer wieder wird diskutiert, dass Mineralöle als schwer abbaubare Fette zur Anreicherung im Körper führen und durch Verschluss der Hautporen die Hautatmung verschlechtern oder die Entwicklung von Akne fördern. Auch Lippenpflegestifte mit Mineralölen sind daher bereits in Kritik geraten.
Trotz der bekanntermaßen guten topischen Verträglichkeit von Paraffinen, besteht ein anhaltendes Interesse nach Zubereitungen, welche Salbengrundlagen enthalten, die sich durch die Gewinnung aus nachwachsenden Rohstoffen auszeichnen. In den Eigenschaften sollen sie denen der Vaseline entsprechen. Bisherige Grundlagen zum Ersatz von Vaseline haben relativ enge Schmelzbereiche, es wurde daher nach möglichen Ersatzgrundlagen mit einem vergleichbaren breiten Schmelzbereich gesucht.

Bereits vor einigen Jahren diente die Abmischung von Bienenwachs und Pflanzenöl als Vaselineersatz. Bienenwachs ist jedoch als natürliches Produkt nicht kurzfristig in großen Mengen herstellbar.

Aus der Internationalen Anmeldung WO 2007/107966 sind Deodorantzubereitungen bekannt, die als Vaselineersatz hydriertes Rizinusöl enthalten, das durch Rizinussamenöl, flüssige Fettalkohole und Pflanzenöle in seiner Viskosität erniedrigt wird. Es wird vermutet, dass aufgrund der höheren Polarität von hydriertem Rizinusöl Duftstoffe länger in diesen kosmetischen Grundlagen verbleiben als in den Vaseline basierten Zubereitungen.
Die chemische Synthese von verzweigtkettigen Alkoholen über die Guerbet-Reaktion ist ein seit langem etabliertes Verfahren in der chemischen Industrie.
Die Kondensation von primären Alkoholen kann z.B. durch Basen katalysiert werden, als Reaktionsprodukt entstehen α-verzweigte Alkohole.

US 3 553 192 A beschreibt eine Salbe aus einem Uridin-Derivat in 85 g weißem Petrolatum und 15 g 2-Octyldodecan-1-ol.

Die Schmelzbereiche von C12-C32-Guerbetalkoholen sind in Goetz Lachmann: "Sasol Olefins & Surfactants ISOFOL C12-C32 Defined Branched Guerbet-Alcohols" 1. Oktober 2003, Seiten 1-12 für kosmetische Produkte beschrieben.

M. Sulzbacher: "The Guerbet reaction of cetyl alcohol", J. Appl. Chem., 1. Januar 1955, Seiten 637-641 beschreibt die Guerbet-Reaktion von Cetylalkohol bei 240 °C unter Verwendung von KOH-Lösungen.

Aus EP 0 970 998 A1 sind Wachsester auf Pflanzenbasis mit vaselineähnlicher Konsistenz bekannt, die aus Guerbet-Alkoholen und Fettsäuren hergestellt werden.

WO 2004/006869 A1 beschreibt Produkte, in denen die Lipidphase einen Schmelzbereich von 25 bis 100 °C aufweist. Diese Lipidphase besteht aus Fettalkoholen, wie z. B. C16/C18-Guerbet-Alkoholen.

US 2011/150805 offenbart Octyldodecanol als Äquivalent von Vaseline, allerdings ohne Schmelzpunktangabe.

Es sind unterschiedliche Guerbetalkohole auf dem Markt, z.B. Eutanol® G/G16 (C16 - C20 Guerbets) von der BASF Personal Care and Nutrition GmbH. Von der Firma Sasol sind unterschiedliche Isofol®-Typen auf dem Markt (z.B. Isofol® C12 bis C32).
Von der Firma Exxon sind verschiedene Exxal™ C16 bis C26 Typen auf dem Markt, vom Jarchem Industries werden z.B. die Jarcol™ C12 bis C36 Typen angeboten. Von der Evonik Goldschmidt GmbH wird z.B. ein Tegosoft® G 20 angeboten.

Einige kurzkettige Guerbetalkoholtypen mit einer Kettenlänge von weniger als 20 Kohlenstoffatomen sind bei Raumtemperatur flüssig. Sie können beispielsweise als kosmetische Emollients eingesetzt werden. Längerkettige C32-C36-Guerbets sind fest und haben einen hohen Schmelzpunkt im Bereich von über 45°C. Hochschmelzende Verbindungen haben in der Regel einen deutlich definierteren Schmelzpunkt und sind somit nicht als Vaselineersatz geeignet. Generell liegt der Schmelzpunkt von verzweigtkettigen Guerbetalkoholen deutlich niedriger als der Schmelzpunkt der entsprechenden linearen Alkohole. [Sasol Olefins & Surfactants, ISOFOL® C12-C32 Defined Branched Guerbet Alcohols, Sasol Germany GmbH, Paul Baumann Straße 1, 45764 Marl, Germany]

### Beschreibung der Erfindung

Es wird ein Gemisch aus Guerbetalkoholen beschrieben, das in der Sensorik und den Anwendungseigenschaften mit Vaseline vergleichbar ist.
Durch Optimierung der Fettalkoholzusammensetzung, Auswahl der Fettalkoholkettenlänge, Durchführung der Guerbetreaktion und gegebenenfalls dem zusätzlichen Einsatz von Diolen als Vernetzer können die Produkteigenschaften des Guerbetalkoholgemisches gezielt eingestellt werden, so dass das Gemisch den Anwendungseigenschaften und der Sensorik von Vaseline vergleichbar ist und einen vergleichbaren Schmelzbereich mit einem ähnlichen Maximum aufweist.

Angaben des Schmelzbereiches von Vaseline in der Literatur liegen zwischen 35 und 60°C. Der Schmelzbereich variiert in Abhängigkeit vom Anteil der kristallinen Bereiche.
Durch Thermische Analyse mittels der Dynamischen Differenzkalorimetrie (DSC - englisch: Differential Scannning Calorimetry) kann jedoch bereits bei niedrigeren Temperaturen der Beginn des Schmelzens gemessen werden.

Die Tatsache, dass das Maximum des Schmelzbereiches bei Vaseline im Bereich der Hauttemperatur liegt und sich Vaseline durch einen relativ breiten Schmelzbereich mit einem langsamen Anstieg ab - 20 °C ± 5 °C bis 70 °C ± 5°C auszeichnet, hat einen wesentlichen Einfluss auf die charakteristischen sensorischen Eigenschaften von Vaseline.
Es wurde daher die Guerbetalkoholmischung ausgewählt, die ein der Vaseline entsprechendes Schmelzverhalten aufweist.

### Charakterisierung des Schmelzbereiches:

Bei der dynamischen Differenzkalorimetrie (DSC) werden Wärmestrom-änderungen gemessen, die aufgrund von temperatur- oder zeitabhängigen Veränderungen der physikalischen und chemischen Struktur des Probenmaterials entstehen. Die DSC erfasst die Wärmeaufnahme des Probenmaterials für das Schmelzen der Probe bei gleichmäßig steigender Heizrate.
Die Ermittlung der Änderung der Wärmemenge kann messtechnisch verschiedenartig erfolgen. Bei der heutigen DSC (Differential Scanning Calorimetry) wird zwischen Wärmestrom-Differenz-Scanning-Kalorimetrie und der leistungskompensierten Differenz-Scanning-Kalorimetrie unterschieden.

Für die Messungen nach der Wärmestrom-Differenz-Scanning-Kalorimetrie (Tabellen 1a und b) wurde das Wärmestrom-DSC Q100 der Firma TA Instruments (Waters GmbH) verwendet.

Es wurden jeweils fünf bis zehn Milligramm des Probenmaterials in Aluminium- Pfännchen eingewogen und hermetisch verkapselt (kaltverschweißt). Diese Pfännchen wurden mit einer Heizrate von 5 K/min einem Temperatur-Programm von - 80°C bis + 100 °C unterworfen und das Schmelzverhalten bzw. Kristallisierverhalten analysiert. Die Ergebnisse wurden reproduzierbar gemessen.

Die Auswertung von Proben mit einer nicht konstanten Basislinie (aufgrund der Temperaturabhängigkeit der Wärmekapazität der Mischungen) und einem sehr breiten Schmelzbereich ist mit üblichen optischen Mitteln durch Ablesen der Werte aus den erhaltenen Diagrammen starken Schwankungen unterworfen, so dass die Temperaturwerte anhand von Enthalpiewerten festgelegt wurden.

Es wurde eine lineare Peak-Auswertung ab - 60°C bis zum Ende des Aufschmelzens (lag zwischen + 40°C und + 80°C liegen) durchgeführt. Die so berechnete Enthalphie wurde prozentual betrachtet. Dadurch können Schmelzbereiche angegeben werden, indem die Temperatur bei 5 % der gesamten Schmelzenthalpie als Startpunkt und bei 99 % der gesamten Schmelzenthalpie als Ende des Bereiches gewählt wurde (Tabelle 1a). Diese Auswahl entsprach den ungefähr aus dem Diagramm optisch ermittelten Werten.

Als Temperaturmaximum wurde der Temperaturwert ermittelt, der sich bei maximalem Peak ergab. Dieser konnte mit ausreichender Genauigkeit aus den Diagrammen abgelesen werden (Tabelle 1b - rechte Spalte).

**Tab.: 1a - Enthalpie - Temperaturaufzeichnung der DSC-Messung**

| | | | | 1% | | **5%** | | 10% | | 50% | | 90% | | 95% | | **99%** | |
|---|---|---|---|---|---|---|---|---|---|---|---|---|---|---|---|---|---|
| | **E in J/g gesamt** | **T (α) in °C** | **T (Ω) in °C** | **E in J/g** | **T in °C** | **E in J/g** | **T in °C** | **E in J/g** | **T in °C** | **E in J/g** | **T in °C** | **E in J/g** | **T in °C** | **E in J/g** | **T in °C** | **E in J/g** | **T in °C** |
| **Vaseline Enzborn** | 64 | -60 | 76 | 1 | -48 | 3 | **-32** | 6 | -17 | 32 | 26 | 57 | 50 | 61 | 56 | 63 | **67** |
| **Vaseline Sigma Aldrich** | 63 | -60 | 76 | 1 | -49 | 3 | **-30** | 6 | -13 | 32 | 29 | 57 | 54 | 60 | 59 | 63 | **67** |
| **Vaseline Hansen** | 73 | -60 | 76 | 1 | -44 | 3 | **-18** | 7 | -5 | 37 | 22 | 66 | 46 | 70 | 55 | 73 | **67** |
| **Vaseline VWR Prolabo** | 90 | -60 | 75 | 1 | -38 | 4 | **-10** | 9 | 0 | 45 | 24 | 81 | 47 | 85 | 56 | 89 | **67** |
| **A 70-30** | 117 | -60 | 67 | 1 | -36 | 6 | **-6** | 12 | 3 | 59 | 19 | 105 | 31 | 111 | 41 | 116 | **60** |
| **B 60-35** | 111 | -60 | 38 | 1 | -31 | 6 | **-10** | 11 | 3 | 56 | 19 | 100 | 30 | 106 | 31 | 110 | **32** |
| **C 60-40** | 125 | -60 | 56 | 1 | -18 | 6 | **-5** | 12 | -1 | 62 | 27 | 112 | 41 | 119 | 42 | 124 | **44** |
| **D 95-5 Ocenol** | 134 | -60 | 59 | 1 | 13 | 7 | **18** | 13 | 21 | 67 | 44 | 121 | 51 | 127 | 52 | 133 | **53** |
| **E 62,5-32,5-5** | 115 | -60 | 52 | 1 | -19 | 6 | **-4** | 12 | 1 | 58 | 22 | 104 | 36 | 109 | 37 | 114 | **39** |

**Tab. 1b: Schmelzbereiche hergestellter Guerbetalkoholmischungen**

| | **Guerbetalkohol-gemisch und Vergleich** | **Gewichtsverhältnis** | **Schmelzbereich °C** | **Schmelzbereich Maximum °C** |
|---|---|---|---|---|
| A | Lanette O / Lorol | 70:30 | - 6 - 60 | 30 |
| B | Lanette O / Lorol | 65:35 | - 10 - 32 | 30 |
| C | Lanette O / Lorol | 60:40 | - 5 - 44 | 40 |
| D | Lanette O / Ocenol 50/55 | 95:5 | 18 - 53 | 50 |
| E | Lanette O / Lorol / Hexandiol | 62,5:32,5:5 | - 4 - 39 | 34 |
| Weisse Vaseline | 4 unterschiedliche | | - 22 - 70 | 45 (Sigma) |
| | Qualitäten: | | | 35 (Enzborn) |
| | Bezug: Sigma Aldrich /Hansen/ Enzborn / VWR Prolabo | | | 25 (Prolabo) |
| | | | | 26 (Hansen) |

Gegenstand der Erfindung sind Guerbetalkoholgemische mit einem durch dynamische Differenzkalorimetrie (DSC) gemessenen Schmelzbereich zwischen -20°C und +70°C, wobei die Breite des Schmelzbereichs mindestens 30 Temperaturgrade umfasst und das Maximum des Schmelzbereichs bei 35 ± 15 °C liegt. Durch Schwankungen der Zusammensetzung von Vaseline insbesondere den unterschiedlichen Anteilen an kristallinen Bereichen variieren die mit der genauen Methode der DSC ermittelten Werte, so dass auch der für die erfindungsgemäßen Guerbetalkoholgemische ermittelte Schmelzbereich im Temperaturbereich zwischen -20 °C und +70 °C, bevorzugt zwischen -15°C und +65°C, besonders bevorzugt zwischen -10 °C und +60 °C und speziell zwischen - 10 °C und 55 °C liegt.

Dabei muss sich der Schmelzbereich nicht über die gesamte Breite hinziehen, er sollte aber mindestens einen Bereich von 30 Temperaturgraden (°C) innerhalb des Temperaturbereiches zwischen -20 °C und +70 °C, bevorzugt innerhalb des Temperaturbereiches zwischen -15°C und +65°C, besonders bevorzugt innerhalb des Temperaturbereiches zwischen -10 °C und +60 °C und speziell innerhalb des Temperaturbereiches zwischen - 10 °C und 55 °C abdecken, vorzugsweise soll er mindestens 40 Temperaturgrade (°C) in seiner Breite ausmachen. Das Maximum des Schmelzbereiches liegt dabei bei 35 ± 15 °C, vorzugsweise bei 35 ± 10 °C und besonders bevorzugt bei 35 ± 5 °C, es fällt somit ungefähr in den Bereich der Hauttemperatur.

Bevorzugt sind daher Guerbetalkoholgemische mit einem Schmelzbereich zwischen -15°C und +65°C, einer Breite des Schmelzbereichs von mindestens 40 Temperaturgraden und einem Maximum bei 35 ± 10 °C.
Besonders bevorzugt sind Guerbetalkoholgemische mit einem Schmelzbereich zwischen - 10°C und +60 °C, einer Breite des Schmelzbereichs von mindestens 40 Temperaturgraden und einem Maximum bei 35 ± 10 °C, sowie speziell
Guerbetalkoholgemische mit einem Schmelzbereich zwischen -15 °C und +55 °C, einer Breite des Schmelzbereichs von mindestens 40 Temperaturgraden und einem Maximum bei 35 ± 5 °C.

Die vorliegende Erfindung betrifft Guerbetalkoholgemische, die erhältlich sind durch Umsetzung von
a) 55 bis 95 Gew. % Cetylstearylalkohol,
b) 5 bis 45 Gew.% unverzweigten, gesättigten Fettalkoholen mit folgender Kettenverteilung:
   C12-Alkohol von 48 - 58 Gew. %
   C14-Alkohol von 18 - 24 Gew. %
   C16-Alkohol von 8 - 12 Gew. %
   C18-Alkohol von 11 - 15 Gew. % und
c) gegebenenfalls 5 Gew. % eines aliphatischen Diols mit mindestens 3 Kohlenstoffatomen, mit der Maßgabe, dass die Gemische einen nach dynamischer Differenzkalorimetrie (DSC) gemessenen Schmelzbereich zwischen -20 °C und +70 °C aufweisen, wobei die Breite des Schmelzbereichs mindestens 30 Temperaturgrade umfasst und das Maximum des Schmelzbereichs bei 35 ± 15 °C liegt,
wobei die Startalkohole (a) bis (c) in einer Guerbet-Reation bis zu einem Umsatz von 60 bis 80% umgesetzt werden.

Bevorzugt sind Guerbetalkoholgemische, die erhältlich sind durch Umsetzung von
a) 60 bis 70 Gew. % Cetylstearylalkohol,
b) 30 bis 40 Gew.% Fettalkoholen mit folgender Kettenverteilung:
   C12-Alkohol von 48 - 58 Gew. %
   C14-Alkohol von 18 - 24 Gew. %
   C16-Alkohol von 8 - 12 Gew. %
   C18-Alkohol von 11 - 15 Gew. % und
c) gegebenenfalls 5 Gew. % eines aliphatischen Diols mit mindestens 3 Kohlenstoffatomen.

Die Komponente a) Cetylstearylalkohol ist ein Gemisch der langkettigen Fettalkohole Hexadecan-1-ol (C16) und Octadecan-1-ol (C18), das als nichtionischer Koemulgator, Emollient und Konsistenzgeber im kosmetischen und pharmazeutischen Bereich eingesetzt wird. Unter der Bezeichnung Lanette® O ist eine Zusammensetzung aus Cetylalkohol und Stearylalkohol mit folgender Kettenverteilung im Handel, die sich besonders für die Herstellung der erfindungsgemäßen Zubereitung eignet:
C16 von 45-55 Gew. % und
C18 von 45-55 Gew. %.

Cetylstearylalkohol hat einen Schmelzbereich von 48-53 °C und ist biologisch abbaubar. Die für die Herstellung des Guerbetalkoholgemisches eingesetzten Fettalkohole weisen eine Kettenlänge von 8 bis 22 Kohlenstoffatomen auf und können gesättigt oder ungesättigt sein. Als ungesättigter Fettalkohol eignet sich beispielsweise Oleylalkohol (Ocenol 50/55), der mit Cetylstearylalkohol im Gewichtsverhältnis 5:95 einen Schmelzbereich von 15 bis 50 °C aufweist.

Erfindungsgemäß werden unverzweigte, gesättigte Fettalkohole mit 12 bis 20 Kohlenstoffatomen eingesetzt. Um den Eigenschaften der Vaseline möglichst weitgehend zu entsprechen hat sich besonders eine Kettenverteilung unverzweigter Fettalkohole von:
C12-alkohol von 48 - 58 Gew. %
C14-alkohol von 18 - 24 Gew. %
C16-alkohol von 8 - 12 Gew. %
C18-alkohol von 11 - 15 Gew. %
bewährt.

Komponente c) kann eingesetzt werden, um die Alkohole weiterhin untereinander zu vernetzen. Diese Vernetzung ermöglicht eine weitere Steuerung und Einstellung des gewünschten Schmelzbereiches.
Die aliphatischen Diole sollten mindestens 3 Kohlenstoffatome aufweisen, bevorzugt wird Hexandiol, besonders bevorzugt Hexan-1,6-diol eingesetzt.

### Synthesebedingungen für Guerbetreaktionen:

Die Guerbetreaktionen werden im Temperaturbereich von 200 bis 260°C durchgeführt, bevorzugt ist ein Temperaturbereich von 220 bis 250° C.

Den zu guerbetisierenden Alkoholen wird dazu eine katalytische Menge an Base, beispielsweise Kalilauge zugesetzt. Gegebenenfalls kommen ergänzend noch CoKatalysatoren wie Zinkoxid oder Übergangsmetalle oder deren Verbindungen zum Einsatz. Die Kettenlänge der Fettalkohole kann dabei breit variiert werden und es können auch Mischungen verschiedener Alkohole gemeinsam guerbetisiert werden. Es werden als Startalkohole natürliche Fettalkohole mit einer Kohlenstoffkettenlänge von 8 bis 22 Kohlenstoffatomen eingesetzt. Unter speziellen Bedingungen, wie erhöhtem Druck oder Auswahl geeigneter Katalysatoren sind auch kürzerkettige Startalkohole einsetzbar. Der Einsatz von ungesättigten Alkoholen ist ebenfalls möglich.
Durch einen Zusatz von Diolen ist es möglich, die entstehenden Guerbetalkohole teilweise zu vernetzen und so eine breitere Molgewichtsverteilung mit den hieraus resultierenden physikalischen Eigenschaften einzustellen. Einsetzbar sind dabei Diole mit mindestens 3 C-Atomen, vorzugsweise 3 bis 18 C-Atomen und besonders bevorzugt wird Hexandiol eingesetzt. Möglich ist auch der Einsatz von Polyolen wie beispielsweise Trimethylolpropan.

Die Guerbetreaktion wird erfindungsgemäß bis zu einem Umsatz der Startalkohole von 60-80 % betrieben. Je nach gewünschtem Eigenschaftsprofil können die verbleibenden Startalkohole dann im Produkt verbleiben oder auch abdestilliert werden.
In den rohen Guerbetalkoholen wird die restliche Katalysator-Base mit einer starken Säure neutralisiert und Salze durch eine Wasserwäsche entfernt. Abschließend kann der pH-Wert mit einer geeigneten Säure wie Milchsäure in den gewünschten, hautneutralen Bereich abgesenkt werden.

Aufgrund der physikalischen, chemischen und insbesondere rheologischen Eigenschaften sind die Mischungen aus Guerbetalkoholen zum Ersatz von Vaseline in kosmetischen oder pharmazeutischen Zubereitungen zu verwenden.
Die erfindungsgemäßen Guerbetalkoholgemische haben einen vergleichbaren Schmelzbereich wie Vaseline, zeigen vergleichbare sensorische Eigenschaften wie Vaseline, dennoch haben sie bessere Anwendungseigenschaften in tensidischen Systemen, da die Schaummenge in Rezepturen im mit Guerbetalkoholgemischen größer ist als in vaselinehaltigen Zubereitungen.

### Kosmetische Zubereitungen

Die erfindungsgemäßen Zubereitungen eignen sich als Basis in allen pharmazeutischen Zubereitungen zur topischen Anwendung und allen kosmetischen Mittel zur Körperpflege und -reinigung wie z.B. Körperöl, Babyöl, Körpermilch, Cremes, Lotionen, sprühbare Emulsionen, Sonnenschutzmittel und Antitranspirantien,. Sie lassen sich besonders in tensidhaltigen Zubereitungen wie z.B. Flüssig- und Stückseifen, Schaum- und Duschbädern, Haarshampoos und Pflegespülungen einsetzen. Möglich ist auch der Einsatz als Pflegekomponente auf Tissues, Papiere, Wipes, Vlies-Produkte, Schwämme, Puffs, Pflaster und Bandagen applizieren, die im Bereich der Hygiene und Pflege verbreitet sind (Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes, Windeln, Taschentücher, Wet Wipes, Hygieneprodukte, Selbstbräunungs Wipes). Sie lassen sich u.a. auch in Zubereitungen zur Haarpflege, Haarreinigung oder Haarfärbung einsetzen. Sie lassen sich weiterhin in Zubereitungen der dekorativen Kosmetik, wie beispielsweise Lippenstiften, lipgloss, Make-up, Foundations, Puder, Lidschatten, Maskara und ähnlichem einsetzen.

Die Einsatzkonzentrationen in den jeweiligen Formulierungen und Zubereitungen entsprechen denen von Vaseline. Die pharmazeutischen und kosmetischen Zubereitungen, enthaltend die erfindungsgemäßen Guerbetalkoholgemische sind daher ebenfalls Gegenstand der Erfindung. Da die Guerbetalkoholgemische insbesondere in tensidischen Zubereitungen Vorteile gegenüber dem Einsatz von Vaseline haben, dadurch dass die Schaummenge höher ist als in vergleichbaren vaselinehaltigen Systemen, sind auch kosmetische und/oder pharmazeutische Zubereitungen enthaltend die erfindungsgemäßen Guerbetalkoholgemische und grenzflächenaktive Substanzen Gegenstand der Erfindung.

Je nach Applikationszweck enthalten die kosmetischen Formulierungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosinaseinhibitoren (Depigmentierungsmittel), Füllstoffe, Hydrotrope, Solubilisatoren, Konservierungsmittel, Parfümöle, Farbstoffe etc., die nachstehend exemplarisch aufgelistet sind.

### Grenzflächen-aktive Substanz b-1)

In einer Ausführungsform der Erfindung enthalten die erfindungsgemäßen Zubereitungen mindestens eine grenzflächen-aktive Substanz. Die erfindungsgemäßen Zubereitungen enthalten den/die grenzflächen-aktiven Substanz(en) in einer Menge von 0 bis 80 Gew.-% , insbesonderen 0 bis 40 Gew.-%, vorzugsweise 0,1 bis 20 Gew.-% vorzugsweise 0,1 bis 15 Gew.-% und insbesondere 0,1 bis 10 Gew.-% bezogen auf das Gesamtgewicht der Zubereitung.

Als grenzflächen-aktive Substanz eignet sich prinzipiell jede Substanz, welche die Oberflächenspannung zwischen der wässrigen und der nicht-wässrigen Phase erniedrigt. Grenzflächen-aktive Substanzen umfassen Emulgatoren und Tenside.

In einer Ausführungsform der Erfindung enthält die erfindungsgemäße Zubereitung mehr als eine grenzflächen-aktive Substanz. Der Fachmann setzt in Abhängigkeit der übrigen Komponenten übliche Systeme (wie z.B. Emulgator und Co-Emulgator) ein.

Ein geeigneter Emulgator ist prinzipiell jede grenzflächen-aktive Substanz, insbesondere jedoch Substanzen mit einem HLB-Wert von 1 bis 20 nach der Griffin Skala. Jedem Emulgator wird ein so genannter HLB-Wert (eine dimensionslose Zahl zwischen 1 und 20, Griffin Skala) zugeschrieben, der angibt, ob eine bevorzugte Wasser- oder Öllöslichkeit vorliegt. Zahlen unter 9 kennzeichnen bevorzugt öllösliche, hydrophobe Emulgatoren, Zahlen über 11 wasserlösliche, hydrophile Emulgatoren. Der HLB-Wert sagt etwas über das Gleichgewicht der Größe und Stärke der hydrophilen und der lipophilen Gruppen eines Emulgators aus.

Die Löslichkeit des Emulgators in den beiden Phasen bestimmt praktisch den Emulsionstyp. Ist der Emulgator besser in Wasser löslich erhält man eine O/W-Emulsion. Hat der Emulgator hingegen eine bessere Löslichkeit in der Ölphase entsteht unter sonst gleichen Herstellungsbedingungen eine W/O-Emulsion.

### Nicht-ionische Emulgatoren

Zur Gruppe der nicht-ionischen Emulgatoren gehören beispielsweise:
(1) Anlagerungsprodukte von 2 bis 50 Mol Ethylenoxid und/oder 1 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe.
(2) C₁₂-C₁₈-Fettsäuremono- und -diester von Anlagerungsprodukten von 1 bis 50 Mol Ethylenoxid an Glycerin.
(3) Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Polyol- und insbesondere Polyglycerinester, wie z. B. Polyolpoly-12-hydroxystearate, Polyglycerinpolyricinoleat, Polyglyceryl-4-Laurate, Polyglycerindiisostearat oder Polyglycerindimerat. Ebenfalls geeignet sind Gemische von Verbindungen aus mehreren dieser Substanzklassen, wie z.B.Polyglyceryl-4 Düsostearate/Polyhydroxystearate/Sebacate
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter C₆-C₂₂-Fettsäuren, Ricinolsäure sowie 12-Hydroxystearinsäure und Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose), oder Mischester, sowie Sucrose Polystearate (kommerziell erhältlich als Emulgade® SUCRO, Cognis GmbH).
(9) Polysiloxan-Polyalkyl-Polyether-Copolymere bzw. entsprechende Derivate.
(10) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. C_{12/18}-Fettsäuremono- und -diester von Anlagerungsprodukten von Ethylenoxid an Glycerin sind als Rückfettungsmittel für kosmetische Zubereitungen bekannt.

Erfindungsgemäß besonders gut geeignete und milde Emulgatoren sind Polyolpoly-12-hydroxystearate und Abmischungen davon, welche beispielsweise unter den Marken "Dehymuls^{®} PGPH" (W/O-Emulgator) oder "Eumulgin^{®} VL 75" (Abmischung mit Coco Glucosides im Gewichtsverhältnis 1:1, O/W-Emulgator) oder Dehymuls^{®} SBL (W/O-Emulgator) von der Cognis Deutschland GmbH vertrieben werden. In diesem Zusammenhang sei insbesondere auf das Europäische Patent **EP 766 661 B1** verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Als lipophile W/O-Emulgatoren eignen sich prinzipiell Emulgatoren mit einem HLB-Wert von 1 bis 8, die in zahlreichen Tabellenwerken zusammengefaßt und dem Fachmann bekannt sind. Für ethoxylierte Produkte lässt sich der HLB-Wert auch nach folgender Formel berechnen: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von C₄-C₆-Polyolen, wie beispielsweise Partialester des Pentaerythrits oder Zuckerestern, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Je nach Formulierung kann es vorteilhaft sein, zusätzlich wenigstens einen Emulgator aus der Gruppe nicht-ionischer O/W-Emulgatoren (HLB-Wert: 8- 18) und/oder Solubilisatoren einzusetzen. Hierbei handelt es sich beispielsweise um die bereits einleitend erwähnten Ethylenoxid-Addukte mit einem entsprechend hohen Ethoxylierungsgrad, z. B. 10 - 20 Ethylenoxid-Einheiten für O/W-Emulgatoren und 20 - 40 Ethylenoxid-Einheiten für sogenannte Solubilisatoren. Erfindungsgemäß besonders vorteilhaft als O/W-Emulgatoren sind Ceteareth-12, Ceteareth-20 und PEG-20 Stearat. Als Solubilisatoren bevorzugt geeignet sind Eumulgin^{®} HRE 40 (INCI: PEG-40 Hydrogenated Castor Oil), Eumulgin^{®} HRE 60 (INCI: PEG-60 Hydrogenated Castor Oil), Eumulgin^{®} L (INCI: PPG-1-PEG-9 Laurylglycolether), sowie Eumulgin^{®} SML 20 (INCI: Polysorbat-20).

Nicht-ionische Emulgatoren aus der Gruppe der Alkyloligoglycoside sind besonders hautfreundlich und daher bevorzugt als O/W-Emulgatoren geeignet. C₈-C₂₂-Alkylmono- und - oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 6 bis 24, vorzugsweise 8 bis 22 C-Atomen. Bezüglich des Glycosidrestes gilt, dass sowohl Monoglycoside, bei denen ein zyklischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare^{®} oder Plantaren® zur Verfügung stehen, enthalten eine glucosidisch gebundene C₈-C,₆-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet. Erfindungsgemäß bevorzugt ist ein Produkt, das unter der Bezeichnung Emulgade^{®} PL 68/50 von der Cognis Deutschland GmbH vertrieben und ein 1:1-Gemisch aus Alkylpolyglucosiden und Fettalkoholen darstellt. Erfindungsgemäß vorteilhaft einsetzbar ist auch ein Gemisch aus Lauryl Glucoside, Polyglyceryl-2-Dipolyhydroxystearate, Glycerin und Wasser, das unter der Bezeichnung Eumulgin^{®} VL 75 im Handel ist.

Als Emulgatoren kommen weiterhin Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Emulgatoren können beispielsweise Silikonemulgatoren enthalten sein. Diese können beispielsweise aus der Gruppe der Alkylmethicon-copolyole und/oder Alkyl-Dimethiconcopolyole gewählt werden, insbesondere aus der Gruppe der Verbindungen, welche gekennzeichnet sind durch die folgende chemische Struktur: 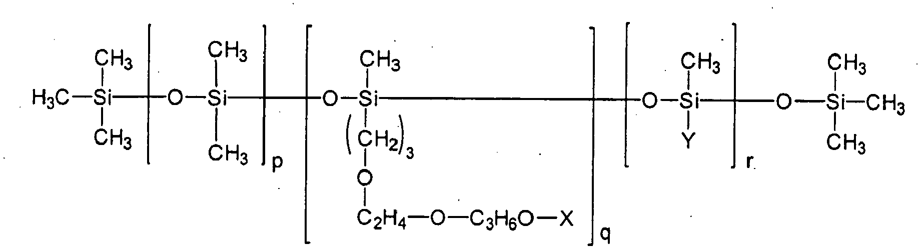 bei welcher X und Y unabhängig voneinander gewählt werden aus der Gruppe H (Wasserstoff) sowie der verzweigten und unverzweigten Alkylgruppen, Acylgruppen und Alkoxygruppen mit 1-24 Kohlenstoffatomen, p eine Zahl von 0-200 darstellt, q eine Zahl von 1-40 darstellt, und r eine Zahl von 1-100 darstellt.

Ein Beispiel für besonders vorteilhaft im Sinne der vorliegenden Erfindung zu verwendenden Silikonemulgatoren sind Dimethiconcopolyole, welche von Evonik Goldschmidt unter den Warenbezeichnungen AXIL® B 8842, ABIL® B 8843, ABIL® B 8847, ABIL® B 8851, ABIL® B 8852, ABIL® B 8863, ABIL® B 8873 und ABIL®B 88183 verkauft werden.

Ein weiteres Beispiel für besonders vorteilhaft im Sinne der vorliegenden Erfindung zu verwendende grenzflächenaktiven Substanzen ist das Cetyl PEG/PPG-10/1 Dimethicone (Cetyl Dimethiconcopolyol), welches von EvonikGoldschmidt unter der Warenbezeichnung ABIL® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopo- lyol, welches von Evonik Goldschmidt unter der Warenbezeichnung ABIL®EM 97 und ABIL®WE 09 verkauft wird.

Weiterhin hat sich als ganz besonders vorteilhaft der Emulgator Lauryl PEG/PPG-18/18 Methicone (Laurylmethiconcopolyol) herausgestellt, welcher unter der Warenbezeichnung Dow Corning® 5200 Formulation Aid von der Gesellschaft Dow Corning Ltd. erhältlich ist. Weiterhin vorteilhaft ist ein Silikonemulgator mit der INCI Bezeichnung Cyclopentasiloxane and PEG/PG-18-18 Dimethicone", der beispsielsweise unter dem Handelsnamen Dow Corning® 5225 C Formulation Aid erhältlich ist.

Ein weiterer vorteilhafter Silikonemulgator ist Octyl Dimethicon Ethoxy Glucosid der Firma Wacker. Für eine erfindungsgemäße Wasser-in-Silikonöl-Emulsion können alle bekannten für diesen Emulsionstyp verwendeten Emulgatoren eingesetzt werden. Erfindungsgemäß besonders bevorzugte Wasser-in-Silikon-Emulgatoren sind dabei Cetyl PEG/PPG- 10/1 Dimethicone und Lauryl PEG/PPG-18/18 Methicone [z.B. ABIL® EM 90 Evonik Goldschmidt), DC5200 Formulation Aid (Dow Corning)] sowie beliebige Mischungen aus beiden Emulgatoren.

Ein geeigneter anionischer O/W Emulgator ist z.B das unter der INCI Bezeichung erhältliche Produkt Disodium Cetearyl Sulfosuccinate (Handelsname Eumulgin® Prisma, Cognis GmbH).

### Tenside

In einer Ausführungsform der Erfindung enthalten die erfindungsgemäßen Zubereitungen als grenzflächen-aktive Verbindungen mindestens ein Tensid. Als grenzflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten.

Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Als **zwitterionische Tenside** werden solche oberflächenaktiven Verbindungen bezeichnet, die im Molekül mindestens eine quartäre Ammoniumgruppe und mindestens eine -COO⁽⁻⁾- oder -SO₃⁽⁻⁾ -Gruppe tragen. Besonders geeignete zwitterionische Tenside sind die so genannten Betaine wie die N-Alkyl-N,N-dimethylammoniumglycinate, beispielsweise das Kokosalkyldimethyl-ammoniumglycinat, N-Acyl-aminopropyl-N,N-dimethylammonium-glycinate, beispielsweise das Kokosacylaminopropyldimethylammoniumglycinat, und 2Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren (beispielsweise unter dem Handelsnamen Dehyton®DC kommerziell erhältlich), N-Hydroxyethyl-N-alkylamidopropylglycine, N-Alkyltaurine, N- Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂-₁₈-Acylsarcosin. Weiterhin geeignten sind Derivate von N-Alkyliminodipropionsäuren, wie beispielsweise N-Lauryl-beta-Iminopropionate, kommerziell erhätlich unter dem Handelsnamen Deriphat® 160 C. Weiterhin geeignet sind Amphoacetate, wie z.B. Cocoamphoacetate (z.B. Dehyton® MC) oder Cocoamphodiacetate (wie z.B. Dehyton® DC).

**Anionische Tenside** sind gekennzeichnet durch eine wasserlöslich machende, anionische Gruppe wie z. B. eine Carboxylat-, Sulfat-, Sulfonat-, Citrat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze. Besonders geeignete anionische Tenside sind Glyceryl Stearate Citrate (wie z.B. kommerziell erhältlich unter den Handelsnamen Imwitor®370, Imwitor® 372P, Axol®C,62 or Dracorin®CE 614035) oder Glyceryl Stearat Lactat Verbindungen. Beispiel für ein geeignetes Alkylsulfat ist Sodium Cetearyl Sulfate (Handelsname Lanette® E), Beispiel für ein geeignetes Phosphat ist Potassium Cetyl Phosphate (Handelsname Amphisol® K). Beispiel für ein geeignetes Acylglutamat ist Sodium Stearoyl Glutamate (Handelsname z.B. Eumulgin® SG). Ein weiteres Beispiel für ein geeignetes anionische Tensid ist Sodium Lauryl Glucose Carboxylate (Handelsname Plantapon® LGC).

Als **kationische Tenside** sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Geeignete pseudo kationische Tenside sind beispielsweise Stearylaminopropyl Dimtheylamine (kommerziell erhältlich unter dem Handelsnamen Dehyquart® S18 oder Incromine® SB oderTegoAmide®S18). Weiterhin können die sehr gut biologisch abbaubaren quaternären Esterverbindungen, wie beispielsweise die unter dem Warenzeichen Stepantex^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden. Unter der Bezeichnung "Esterquats" werden im allgemeinen quaternierte Fettsäuretri-ethanolaminestersalze verstanden. Sie können den erfindungsgemäßen Zubereitungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar. Geeignete kationische Tenside sind beispielsweise Dipalmitoylethyl Hydroxyethylmonium Methosulfate (Handelsname Dehyquart®C4046), Distearoylethyl Hydroxyethylmonium Methosulfate (Handelsname Dehyquart®F75), Dicocoylethyl Hydroxyethylmonium Methosulfate (Handelsname Dehyquart® L80), Behentrimonium Chloride (Handelsname Varisoft® BT), Distearyldimonium Chloride (Handelsname Varisoft® TA 100), Palmitamidopropyltrimonium Chloride (Handelsname Varisoft® PATC).

### Wachskomponente b-2)

In einer Ausführungsform der Erfindung enthalten die erfindungsgemäßen Zubereitungen mindestens eine Wachskomponente. Die erfindungsgemäßen Zubereitungen enthalten den/die Wachskomponente(n) in einer Menge von 0 bis 40 Gew.-%, insbesondere von 0 bis 20 Gew.-%, vorzugsweise 0,1 bis 15 Gew.-% und insbesondere 0,1 bis 10 Gew.-% bezogen auf das Gesamtgewicht der Zubereitung.

Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30 C oder darüber schmelzen.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind so genannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter, Shea Butter und Kokosfett.

Geeignet sind u.a. die Dreifachester von Glycerin mit C12-C60-Fettsäuren und insbesondere C12-C36-Fettsäuren. Hierzu zählt gehärtetes Rizinusöl, ein Dreifachester aus Glycerin und einer Hydroxystearinsäure, der beispielsweise unter der Bezeichnung Cutina HR im Handel ist. Ebenso geeignet sind Glycerintristearat, Glycerintribehenat (z. B. Syncrowax HRC), Glycerintripalmitat oder die unter der Bezeichnung Syncrowax HGLC bekannten TriglyceridGemische, mit der Vorgabe, dass der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis Deutschland GmbH & Co. KG vermarkteten Produkte Novata AB und Novata B (Gemisch aus C12-C18-Mono-, Di- und Triglyceriden) sowie Cutina® HVG (Hydrogenated Vegetable Glycerides) oder Cutina® GMS (Glycerylstearat).

Zu den erfindungsgemäß als Wachskomponente einsetzbaren Fettalkoholen zählen die C12-C50-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß besonders bevorzugt geeignet sind C14-C22-Fettalkohole, die beispielsweise von der Cognis Deutschland GmbH unter der Bezeichnung Lanette 16 (C16-Alkohol), Lanette 14 (C14-Alkohol), Lanette O (C16/C18-Alkohol) und Lanette 22 (C18/C22-Alkohol) vermarktet werden. Fettalkohole verleihen den Zubereitungen ein trockeneres Hautgefühl als Triglyceride und sind daher gegenüber letzteren bevorzugt.

Als Wachskomponenten können auch C14-C40-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C16-C40-Alkylstearate, C20-C40-Alkylstearate (z. B. Kesterwachs K82H), C20-C40-Dialkylester von Dimersäuren, C18-C38-Alkylhydroxystearoylstearate oder C20-C40-Alkylerucate. Ferner sind C30-C50-Alkylbienenwachs, Tristearylcitrat, Trüsostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar.

### Polymere b-3)

In einer Ausführungsform der Erfindung enthalten die erfindungsgemäßen Zubereitungen mindestens ein Polymer. Die erfindungsgemäßen Zubereitungen enthalten das/die Polymere(n) in einer Menge von 0 bis 20 Gew.-%, vorzugsweise 0,05 bis 18 Gew.-% vorzugsweise 0,05 bis 15 Gew.-%, besonders bevorzugt 0,05 bis 10 Gew.-%, insbesondere 0,1 bis 1 Gew.-% bezogen auf das Gesamtgewicht der Zubereitungen. In einer bevorzugten Ausführungsform der Erfindung enthalten die erfindungsgemäßen Zubereitungen das Polymer/die Polymere in einer Menge von 0,1 bis 5 Gew.-%, insbesondere 0,1 bis 3 Gew.-%, insbesondere 0,1 bis 2 Gew.-% bezogen auf das Gesamtgewicht der Zubereitung. Geeignete **kationische Polymere** sind beispielsweise kationische Cellulosederivate, wie z. B. eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat^{®} (BASF), Kondensationsprodukte von Polyglycolen und Aminen, quaternierte Kollagenpolypeptide, wie beispielsweise Lauryldimonium hydroxy-propyl hydrolyzed collagen (Lamequat^{®}L/Grünau), quaternierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine^{®}/Sandoz), Copolymere der Acrylsäure mit Dimethyldiallylammoniumchlorid (Merquat^{®} 550/Chemviron), Polyaminopolyamide, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z. B. Mirapol^{®} A-15, Mirapol^{®} AD-1, Mirapol^{®} AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Besonders geeignete anionische Polymer sind solche mit der INCI Bezeichnung Carbomer, wie z.B die Carbopol Typen 980, 980,981,1382,2984,5984 sowie die unter den Handelsnamen Rheocare®C plus and Rheocare®400 erhältlichen Produkte. Weiterhin geeignete anionische Polymere sind solche mit dem INCI Namen Acrylates/C10-30 Alkyl Acrylate Crosspolymer (Handelsnamen z.B. Pemulen®TR , Pemulen® TR 2, Carbopol®Ultrez), Acrylates Copolymer (Handelsnamen z.B. Rheocare TTA, TTN, TTN-2), Acrylamide/Sodium Acrylate Copolymer (Handelsnamen z.B. Cosmedia®ATC), Sodium Polyacrylate (Handelsnamen z.B. Cosmedia® ATH, Cosmedia®SP), Polyacrylamides (Handelsnamen z.B. Sepigel® 305 or Sepigel® 501). Bevorzugte anionische Polymere sind Polyacrylsäure Homo- und Copolymere.

Weiterhin geeignete Polymere sind Silicone Elastomer Gums, wie z.B. Silikone Elastomer Gemische, wie z.B. Gemische mit den INCI Bezeichnungen Cycolpentasiloxane (and) Dimethiconol (and) Dimethicone Crosspolymer (Handelsname Dow Corning®DC 9027), Gemische mit der INCI Bezeichnung Isodecyl neopentanoate (and) Dimethicone / bisisobutyl PPG-20 Crosspolymer (Handelsname Dow Corning®DC EL 8051 IN), Gemische mit der INCI Bezeichnung Dimethicone / Vinyl Dimethicone Crosspolymer (and) C12-14 Pareth-12) (Handelsname Dow Corning®DC 9509) sowie Gemische mit der INCI Bezeichnung Dimethicone / Vinyl Dimethicone Crosspolymer (and) Silica (Handelsname Dow Corning®DC 9701 Cosmetic Powder).

Als Polymere eigen sich ebenso Polysaccharide, insbesondere Xanthan-Gum, Guar-Gum, Agar-Agar, Alginate und Tylosen sowie Tara Gum, Carraghenan, Sclerotium Gum und natürliche Cellulose.

### Weitere Ölkörper b-4)

Körperpflegemittel, wie Cremes, Körperöle, Lotionen und Milchen, enthalten üblicherweise eine Reihe weiterer Ölkörper und Emollients, die dazu beitragen, die sensorischen Eigenschaften weiter zu optimieren. Die Ölkörper (erfindungsgemäße Verbindungen plus weitere Ölkörper) sind üblicherweise in einer Gesamtmenge von 0,1 - 80, insbesondere 0,5 bis 70, bevorzugt 1 bis 60, insbesondere 1 bis 50 Gew.-%, insbesondere 1 bis 40 Gew.-%, vorzugsweise 5 - 25 Gew.-% und insbesondere 5 - 15 Gew.-% enthalten. Die weiteren Ölkörper sind üblicherweise in einer Menge von 0,1 bis 40 Gew.-% enthalten

Als weitere Ölkörper kommen beispielsweise Guerbetalkohole auf Basis von Fettalkoholen mit 6 bis 18, vorzugsweise 8 bis 10 Kohlenstoffatomen, in Frage sowie Ester wie Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol), Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylylether (Cetiol® OE), Ringöffnungsprodukte von epoxidierten Fettsäureestern mit Polyolen und Kohlenwasserstoffen oder deren Gemischen. Weiterhin geeignet sind Ester von 2-Propylheptanol mit n-Octansäure, wie z.B. kommerziell erhältlich unter dem Handelsnamen Cetiol®SenSoft (Cognis GmbH). Weiterhin geeignet sind Kohlenwasserstoffe, wie zum Beispiel Undecan und Tridecan. Weiterhin geeignet sind Alkane, wie z.B. die Gemische mit der INCI Bezeichnung Conocnut/Palm/Palm Kernel Oil Alkanes (Handelsname Vegelight 1214 der Fa. Biosynthesis).

Überraschenderweise wurde gefunden, dass sich die erfindungsgemäßen Verbindungen insbesondere eignen, um öllösliche kristalline UV-Lichtschutzfilter zu solubilisieren.

Ein Gegenstand der Erfindung betrifft Zubereitungen, enthaltend wenigstens eine Verbindung gemäß Anspruch 1 und mindestens einen UV-Lichtschutzfilter, vorzugsweise einen öllöslichen UV-Lichtschutzfilter.

Erfindungsgemäß sind als UV-Lichtschutzfilter bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
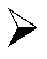 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben
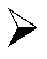 3-(4'-Trimethylammonium) benzyliden- bornan-2-on-methylsulfat (Mexoryl SO)
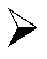 3,3'-(1,4-Phenylendimethin)-bis (7,7- dimethyl-2-oxobicyclo-[2.2.1] heptan-1-methansulfonsäure) and salts (Mexoryl SX)
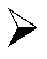 3-(4'-Sulfo)-benzyliden-bornan-2-on and salts (Mexoryl SL)
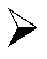 Polymer von N-{(2und 4)- [2-oxoborn-3-yliden)methyl}benzyl]acrylamid (Mexoryl SW)
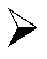 2-(2H-Benzotriazol-2-yl)-4-methyl-6-(2-methyl-3-(1,3,3,3-tetramethyl-1-(trimethylsilyloxy) disiloxanyl)propyl) phenol (Mexoryl SL)
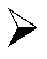 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
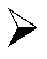 Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene);
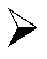 Ester der Salicylsäure, vorzugsweise Salicylsäure-2-ethylhexylester, Salicylsäure-4-isopropylbenzylester, Salicylsäurehomomenthylester;
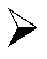 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxy-benzophenon;
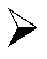 Ester der Benzalmalonsäure, vorzugsweise 4-Methoxybenzmalonsäuredi-2-ethylhexylester;
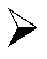 Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl) anilino]-1,3,5-triazin (Uvinul T 150) wie in der EP 0818450 A1 beschrieben oder 4,4'-[(6-[4-((1,1-Dimethylethyl)amino-carbonyl) phenyl-amino]-1,3,5-triazin-2,4- diyl)diimino] bis(benzoesäure-2- ethylhexylester) (Uvasorb® HEB);
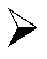 2,2(-Methylen-bis(6-(2H-benzotriazol-2-yl)-4- (1,1,3,3-tetramethyl-butyl)phenol) (Tinosorb M);
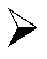 2,4-Bis[4-(2-ethylhexyloxy)-2- hydroxyphenyl]-6-(4- methoxyphenyl)-1,3,5- triazin (Tinosorb S);
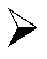 Propan-1,3-dione, wie z.B. 1-(4-tert.Butylphenyl)-3-(4'methoxyphenyl)propan-1,3-dion;
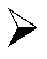 Ketotricyclo(5.2.1.0)decan-Derivate, wie in der EP 0694521 B1 beschrieben;
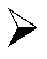 Dimethicodiethylbenzalmalonate (Parsol SLX).

Als wasserlösliche UV - Filter kommen in Frage:
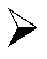 2-Phenylbenzimidazol-5-sulfonsäure und deren Alkali-, Erdalkali-, Ammonium-, Alkylammonium-, Alkanolammonium- und Glucammoniumsalze;
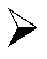 2,2(-(1,4-Phenylen)bis(1H-benzimidazol- 4,6-disulfon-säure, Mononatriumsalz) (Neo Heliopan AP)
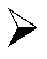 Sulfonsäurederivate von Benzophenonen, vorzugsweise 2-Hydroxy-4-methoxybenzophenon-5-sulfonsäure und ihre Salze;
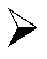 Sulfonsäurederivate des 3-Benzylidencamphers, wie z.B. 4-(2-Oxo-3-bornylidenmethyl)-benzolsulfonsäure und 2-Methyl-5-(2-oxo-3-bornyliden)sulfonsäure und deren Salze.
In einer bevorzugten Ausführungsform der Erfindung enthalten die Zubereitungen mindestens einen öllöslichen UV-Lichtschutzfilter sowie mindestens einen wasserlöslichen UV-Lichtschutzfilter.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Die erfindungsgemäßen Zubereitungen können auch unlösliche Lichtschutzpigmente, nämlich feindisperse Metalloxide bzw. Salze enthalten. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. -Carotin, -Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

In einer bevorzugten Ausführungsform der Erfindung enthalten die Zubereitungen mindestens einen UV-Lichtschutzfilter ausgewählt aus der Gruppe bestehend aus 4-Methybenzylidene Camphor, Benzophenone-3, Butyl Methoxydibenzoylmethane, Bis-Ethylhexyloxyphenol Methoxyphenyl Triazine, Methylene Bis-Benzotriazolyl Tetramethylbutylphenol, Diethylhexyl Butamido Triazone, Ethylhexyl Triazone und Diethylamino Hydroxybenzoyl Hexyl Benzoate, 3-(4'-Trimethylammonium) benzyliden-bornan-2-on-methylsulfat, 3,3'-(1,4-Phenylendimethin)-bis(7,7-dimethyl-2-oxobicyclo-[2.2.1]heptan-1-methansulfonsäure) und ihre Salze, 3-(4'-Sulfo)-benzyliden-bornan-2-on und ihre Salze, Polymer von N-{(2und 4)- [2-oxoborn-3-yliden)methyl}benzyl]acrylamid, 2-(2H-Benzotriazol-2-yl)-4-methyl-6-(2-methyl-3-(1,3,3,3-tetramethyl-1-(trimethylsilyloxy)disiloxanyl)propyl)phenol, Dimethicodiethyl benzalmalonate und ihren Mischungen.

Diese UV-Lichtschutzfilter sind beispielsweise unter den folgenden Handelsnamen kommerziell erhältlich:
NeoHeliopan®MBC (INCI: 4-Methylbenzylidene Camphor; Hersteller: Symrise); NeoHeliopan® BB (INCI: Benzophenone-3, Hersteller: Symrise); Parsol®1789 (INCI: Butyl Methoxydibenzoylmethane, Hersteller: Hoffmann-La Roche (Givaudan); Tinosorb®S (INCI: Bis-Ethylhexyloxyphenol Methoxyphenyl Triazine); Tinosorb®M (INCI: Methylene Bis-Benzotriazolyl Tetramethylbutylphenol): Herstelller: Ciba Specialty Chemicals Corporation; Uvasorb®HEB (INCI: Diethylhexyl Butamido Triazone, Hersteller: 3V Inc.), Uvinul®T 150 (INCI: Ethylhexyl Triazone, Hersteller: BASF AG); Uvinul® A plus (INCI: Diethylamino Hydroxybenzoyl Hexyl Benzoate: Hersteller: BASF AG; Mexoryl® SO: 3-(4'-Trimethylammonium) benzyliden- bornan-2-on-methylsulfat, INCI: Camphor Benzalkonium Methosulfate; Mexoryl®SX: 3,3'-(1,4-Phenylendimethin)-bis (7,7- dimethyl-2-oxobicyclo-[2.2.1] heptan-1-methansulfonsäure), CTFA: INCI Terephthalylidene Dicamphor Sulfonic Acid; Mexory® SL: 3-(4'-Sulfo)-benzyliden-bornan-2-on, INCI Benzylidene Camphor Sulfonic Acid; Mexoryl®SW: Polymer von N-{(2und 4)-[2-oxoborn-3-yliden)methyl}benzyl]acrylamid, INCI Polyacrylamidomethyl Benzylidene Camphor; Mexoryl®SL: 2-(2H-Benzotriazol-2-yl)-4-methyl-6-(2-methyl-3-(1,3,3,3-tetramethyl-1-(trimethylsilyloxy) disiloxanyl)propyl) phenol; INCI: DROMETRIZOLE TRISILOXANE; Parsol® SLX: Dimethicodiethylbenzalmalonate, INCI Polysilicone-15.

Die erfindungsgemäßen Zubereitungen können die UV-Lichtschutzfilter in Mengen von 0,5 bis 30 Gew.-%, vorzugsweise 2,5 bis 20 Gew.-%, besonders bevorzugt 5 - 15 Gew.-% - bezogen auf die Zubereitung - enthalten.

### Weitere Inhaltsstoffe

Als **Verdickungsmittel** eignen sich beispielsweise Aerosil-Typen (hydrophile Kieselsäuren), Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox). Ein geeigneter Verdicker ist beispielsweise das unter den Handelsnamen Cosmedia® Gel CC erhältliche Produkt mit der INCI Bezeichnung Dicaprylyl Carbonate, Stearalkonium Hectorite and Propylene Carbonate. Unter **biogenen Wirkstoffen** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen. **Desodorierende Wirkstoffe / Antiperspirantien** wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker. Als **Insekten-Repellentien** kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage. Als **Selbstbräuner** eignet sich Dihydroxyaceton oder Erythrulose. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage. Als **Konservierungsmittel** eignen sich beispielsweise Phenoxyethanol, Formaldehydlösung, Parabene, Pentandiol, Chlorphenesin, Caprylylglykol, Ethylhexylglycerine oder Sorbinsäure sowie die unter der Bezeichnung Surfacine® bekannten Silberkomplexe und die in Anlage 6, Teil A und B der Kosmetikverordnung aufgeführten weiteren Stoffklassen. Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage. Als **Perlglanzwachse oder Perlglanz-Verbindungen,** insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Stearyl Citrate, Cyclodextrin, Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen. Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Ein geeignetes Überfettungsmittel ist beispielsweise die Mischung von Cocoglucosiden und Glyceryl Oleate (kommerziell erhältlich als Lamesoft® PO65 von Cognis GmbH).

Geeignete Füllstoffe sind Substanzen, die beispielsweise die sensorischen oder kosmetischen Eigenschaften einer Zubereitung verbessern und die beispielsweise ein samtiges oder seidiges Gefühl erzeugen oder verstärken (sog. Skin-Sensory Modifier). Geeignete Füllstoffe sind Stärke und Stärkederivate (wie z.B. Tapioka Stärke, Aluminium Starch Octenyl Succinate, Sodium Octenyl Succinat, Distärke Phosphat), Pigmente, die nicht hauptsächlich als UV-Filter oder Farbstoffe dienen (wie z.B. Bornitrid) und/oder Aerosil^{®} (CAS-Nr. 7631-86-9), und/oder Talkum, sowie beispielsweise Polymethyl Methacrylate (z.B. Cosmedia^{®} PMMA V8/V12), Silica (z. B. Cosmedia^{®} SILC), Stearalkonium Hectorite (wie im kommerziell erhältlichen Produkt Cosmedia® Gel CC enthalten) sowie HDI/Trimethylol Hexyllactone Crosspolymer (wie im kommerziell erhältlichen Produkt Cosmedia^{®} CUSHION enthalten).

Als **Stabilisatoren** können Metallsalze von Fettsäuren, wie z. B. Magnesium-, Aluminium- und/oder Zinkstearat bzw. -ricinoleat eingesetzt werden. Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

Die erfindungsgemäßen Zubereitungen sowie die Verbindung gemäß Anspruch 1 eignen sich insbesondere in kosmetischen und/oder pharmazeutischen Zubereitungen zur Benetzung oder Imprägnierung oder Beschichtung von Gebrauchs- und Hygienetüchern, die zur Körperreinigung und/oder zur Körperpflege eingesetzt werden.

Als Gebrauchs- und Hygienetücher seien exemplarisch genannt: Tissues, Papiere, Wipes, Vlies-Produkte, Schwämme, Puffs, Pflaster und Bandagen, die ihren Einsatz im Bereich der Hygiene und Pflege finden. Dies können sein Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes, Windeln, Taschentücher, Wet Wipes, Hygieneprodukte sowie Selbstbräunungs-Wipes.

### Beispiele

### (1) Herstellbeispiele

### Synthese der Guerbet-Alkohole im Labor

Nachfolgende Synthesen wurden mit folgenden Spezifikationen durchgeführt: Lanette O - Cetylstearylalkohol mit Kettenverteilung C16 von 45-55% und C18 von 45-55% Lorol - "Lorol technisch", C12-18 Fettalkohol mit folgender Kettenverteilung:
C12 48 - 58%
C14 18-24%
C16 8 - 12%
C18 11 - 15%

### A) Beispielsynthese Lanette O / Lorol 70:30

2700 g Lanette O wurden mit 1160 g Lorol vermischt und zu der Schmelze wurden 0,08 g Zinkoxid sowie portionsweise 70 g 50%ige, wässrige Kalilauge zugegeben.

Die Mischung wurde unter partiellem Rückfluss, bei dem die Alkohole zurückgehalten wurden, das Reaktionswasser aber entweichen konnte, auf zunächst 220°C erhitzt. Nach einigen Stunden wurde die Temperatur schrittweise auf 250°C erhöht.

Sobald der Zielumsatz erreicht worden war, wurde der Ansatz ein Mal mit Wasser gewaschen und die verbliebene Alkalimenge mit Milchsäure neutralisiert, im Vakuum getrocknet und mit Filterhilfsmittel über einen Tiefenfilter filtriert.

### B) Beispielsynthese Lanette O / Lorol 65:35

845 g Lanette O wurden mit 455 g Lorol vermischt und zu der Schmelze wurden 0,025g Zinkoxid sowie portionsweise 22 g 50%ige, wässrige Kalilauge zugegeben.

Die Mischung wurde unter partiellem Rückfluss, bei dem die Alkohole zurückgehalten wurden, das Reaktionswasser aber entweichen konnte, auf zunächst 220°C erhitzt. Nach einigen Stunden wurde die Temperatur schrittweise auf 250°C erhöht.

Sobald der Zielumsatz erreicht worden war, wurde der Ansatz einmal mit Wasser gewaschen und die verbliebene Alkalimenge mit Milchsäure neutralisiert, im Vakuum getrocknet und mit Filterhilfsmittel über einen Tiefenfilter filtriert.

### C) Beispielsynthese Lanette O / Lorol 60:40

300 g Lanette O wurden mit 200 g Lorol vermischt und zu der Schmelze wurden 0,01 g Zinkoxid sowie portionsweise 10 g 50%ige, wässrige Kalilauge zugegeben.

Die Mischung wurde unter partiellem Rückfluss, bei dem die Alkohole zurückgehalten wurden, das Reaktionswasser aber entweichen konnte, auf zunächst 220°C erhitzt. Nach einigen Stunden wurde die Temperatur schrittweise auf 250°C erhöht.

Sobald der Zielumsatz erreicht worden war, wurde der Ansatz ein Mal mit Wasser gewaschen und die verbliebene Alkalimenge mit Milchsäure neutralisiert, im Vakuum getrocknet und mit Filterhilfsmittel über einen Tiefenfilter filtriert.

### D) Beispielsynthese Lanette O / Ocenol 95 : 5

1235 g Lanette O wurden mit 65 g HD Ocenol 60/65 vermischt und zu der Schmelze wurden 0,025 g Zinkoxid sowie portionsweise 22 g 50%ige, wässrige Kalilauge zugegeben.

Die Mischung wurde unter partiellem Rückfluss, bei dem die Alkohole zurückgehalten wurden, das Reaktionswasser aber entweichen konnte, auf zunächst 220°C erhitzt. Nach einigen Stunden wurde die Temperatur schrittweise auf 250°C erhöht.

Sobald der Zielumsatz erreicht worden war, wurde der Ansatz ein Mal mit Wasser gewaschen und die verbliebene Alkalimenge mit Milchsäure neutralisiert, im Vakuum getrocknet und mit Filterhilfsmittel über einen Tiefenfilter filtriert.

### E) Beispielsynthese Lanette O / Lorol / Hexandiol 62,5:32,5:5

312,5 g Lanette O wurden mit 162,5 g Lorol und 25 g Hexan-1,6-diol vermischt und zu der Schmelze wurden 0,08g Zinkoxid sowie portionsweise 80 g 50%ige, wässrige Kalilauge zugegeben.

Die Mischung wurde unter partiellem Rückfluss, bei dem die Alkohole zurückgehalten wurden, das Reaktionswasser aber entweichen konnte, auf zunächst 220°C erhitzt. Nach einigen Stunden wurde die Temperatur schrittweise auf 250°C erhöht.

Sobald der Zielumsatz erreicht worden war, wurde der Ansatz einmal mit Wasser gewaschen und die verbliebene Alkalimenge mit Milchsäure neutralisiert, im Vakuum getrocknet und mit Filterhilfsmittel über einen Tiefenfilter filtriert.

### (2) Formulierung und Sensorik

A) Die Anwendungseigenschaften der Guerbetalkohole wurden in einer kosmetischen Formulierung mit 1 Gew. % Cosmedia® SP (Natriumpolyacrylat), 10 Gew. % Cetiol® LC (Coco-Caprylat/Caprat) und 3 Gew. % Glycerin untersucht. Die Einsatzkonzentration an Guerbetalkoholen bzw. Vaseline betrug jeweils 6 Gew. %.

**Tab. 2.1 Formulierung:**

| | **V** | **1** | **2** | **3** |
|---|---|---|---|---|
| | | | | |
| COSMEDIA® SP (Natriumpolyacrylat) | 1,0 | 1,0 | 1,0 | 1,0 |
| CETIOL® LC (Coc-Caprylat / Caprat) | 10,0 | 10,0 | 10,0 | 10,0 |
| Vaseline, weiss (Sigma Aldrich) | **6,0** | -- | -- | -- |
| Guerbetalkoholgemisch A (70:30) | -- | **6,0** | -- | -- |
| Guerbetalkoholgemisch B (65:35) | | | **6,0** | |
| Guerbetalkoholgemisch E | | | | **6,0** |
| Glycerol | 3,0 | 3,0 | 3,0 | 3,0 |
| Wasser, demin. | 79,9 | 79,9 | 79,9 | 79,9 |
| Euxyl K 100 (Konservierungsmittel) | 0,1 | 0,1 | 0,1 | 0,1 |
| pH-Wert | 6,1 | 6,4 | 6,0 | 6,0 |
| Viskosität (Brookfield, RVF, 23°C, Spindel TE, 4 UpM, mit Helipath) mPa*s | 162500 | 125000 | 162500 | 125000 |

### Sensorik im Vergleich zu Vaseline in einer kosmetischen Formulierung:

Die Sensorik der Formulierung wurde von 5 Testpersonen nach definierten Kriterien bewertet. Vaseline stellt den Standard im Vergleich dar (+ beschreibt das Urteil einer Testperson). Die Formulierung mit dem Guerbetalkoholgemisch B (63:35) zeigt vergleichbare sensorische Eigenschaften wie die Vaseline-Formulierung. Insbesondere zieht es im Vergleich zum Standard etwas schneller in die Haut ein und wird als weniger ölig und wachsartig empfunden.

**Tab. 2.2 Ergebnisse der sensorischen Untersuchung**

| | **-** | **Standard** | **+** | |
|---|---|---|---|---|
| Spreitung (gering) | | +++++ | | Spreitung (hoch) |
| Absorption 1 min (langsam) | | +++ | ++ | Absorption 1 min (schnell) |
| Absorption 3 min (langsam) | | ++++ | + | Absorption 3 min (schnell) |
| Rückstände (viel) | | ++++ | + | Rückstände (wenig) |
| Klebrigkeit (stark) | | ++++ | + | Klebrigkeit (gering) |
| Öligkeit (stark) | | +++ | ++ | Öligkeit (gering) |
| Wachsartigkeit (stark) | + | ++ | ++ | Wachsartigkeit (gering) |
| Samtigkeit (gering) | | ++++ | + | Samtigkeit (ausgeprägt) |
| Seidigkeit (gering) | | +++++ | | Seidigkeit (ausgeprägt) |
| Pulvergefühl (gering) | | +++++ | | Pulvergefühl (ausgeprägt) |
| Weichheit (gering) | + | ++ | ++ | Weichheit (ausgeprägt) |
| Glätte (gering) | + | +++ | + | Glätte (ausgeprägt) |
| Pflegegefühl (gering) | + | ++ | ++ | Pflegegefühl (ausgeprägt) |
| Akzeptanz (gering) | | ++++ | + | Akzeptanz (hoch) |

### (3) Einsatz in Tensidsystemen

Die Anwendungseigenschaften der Guerbetalkohole wurden in einer tensidischen Formulierung mit 16,1 % Texapon® N70 (Natriumlaurethsulfat 2EO), 11,1 % Dehyton® PK45 (Cocamidopropylbetain), 2,15 % Comperlan® CMEA (Cocamide MEA), 4% Sonnenblumenöl, 4% Edenor® C12 (Laurinsäure), 0,2 % Dehyquart® GUAR N (Guarhydroxypropyltrimoniumchlorid), 0,2 % EDTA BD, 0,5 % Glycerin, 0,5 % NatriumBenzoat und 1,2 % Zitronensäure untersucht. Die Einsatzkonzentration an Guerbetalkoholen bzw. Vaseline betrug jeweils 4 Gew. %.

**Tab. 3.1 Rezeptur:**

| | **4** | **5** |
|---|---|---|
| TEXAPON® N70 | 16,1 | 16,1 |
| DEHYTONL® PK45 | 11,1 | 11,1 |
| COMPERLAN® CMEA | 2,15 | 2,15 |
| Sonnenblumenöl | 4,0 | 4,0 |
| Edenor C 12 | 4,0 | 4,0 |
| Vaseline, weiss (Sigma Aldrich) | -- | **4,0** |
| Guerbetalkoholgemisch A (65:35) | **4,0** | -- |
| DEHYQUART® GUAR N | 0,2 | 0,2 |
| EDTA BD | 0,2 | 0,2 |
| Glycerol | 0,5 | 0,5 |
| Natriumbenzaot | 0,5 | 0,5 |
| Wasser, demin. | 56,05 | 56,55 |
| Zitronensäure (50%) | 1,2 | 0,7 |
| pH-Wert | 4,6 | 4,9 |
| Viskosität (Brookfield, RVF, 23°C, Spindel 5, 10 UpM) mPa*s | 13400 | 14400 |

### Schäumungseigenschaften

Die Schäumungseigenschaften wurden in einem Sita Rotorfoam Messgerät in einer 1 Gew. %igen Lösung bei 15°dH bei 30°C bestimmt. Das Schaumverhalten der Guerbet-Formulierung, insbesondere das Anschäumen nach ca. 30 Sekunden, ist vergleichbar zur Formulierung mit Vaseline. Im weiteren Verlauf der Messung entwickelt die Testrezeptur auf Guerbet-basis eine größere Schaummenge als die Vaseline-Formulierung. Somit bieten die untersuchten neuen Guerbet-Alkohole vergleichbare oder bessere Anwendungseigenschaften als Vaseline.

**Tab. 3.2 Schäumungseigenschaften**

| | **Schaumhöhe in ml** | |
|---|---|---|
| **Zeit [s]** | **4 mit Guerbetalkoholgemisch A** | **mit Vaseline** |
| 30 | 138 | 126 |
| 60 | 155 | 134 |
| 90 | 190 | 139 |
| 120 | 208 | 150 |
| 150 | 218 | 154 |
| 180 | 229 | 156 |
| 210 | 241 | 149 |
| 240 | 242 | 150 |
| 270 | 253 | 150 |
| 300 | 261 | 126 |

### (4) Okklusivität

Über eine Bestimmung des TEWL (Transepidermaler Wasserverlust) wurde die Okklusivität der Guerbet-Alkohole bestimmt.
Die Okklusivwirkung wurde über die Reduktion der Wasserdurchlässigkeit der Haut mit Hilfe eines Evaporimeter-Verfahrens bestimmt. Dazu wurde der Wasserdampfgradient über der ölbehandelten bzw. unbehandelten Haut des Unterarms mit zwei Messsonden in klimatisierter Umgebung gemessen und daraus die Wasserdurchlässigkeit der Haut ermittelt.

Vaseline wurde als Positiv-Standard, IPM als Negativ-Standard verwendet. Im Folgenden die Einordnung der Proben hinsichtlich der Okklusivität:
1. Vaseline ausgeprägt bis stark okklusiv
2. Guerbetalkoholgemisch B - mäßig bis stark okklusiv
3. Guerbetalkoholgemisch D - mäßig bis stark okklusiv
4. IPM - CE92010016 ausgeprägt bis wenig okklusiv

Beide untersuchten Guerbetalkohole zeigen stark okklusive Eigenschaften und eignen sich somit gut als Vaseline-Ersatz geeignet.

## Patentansprüche

1. Guerbetalkoholgemisch, erhältlich durch Umsetzung von
a) 55 bis 95 Gew. % Cetylstearylalkohol,
b) 5 bis 45 Gew.% unverzweigten, gesättigten Fettalkoholen mit folgender Kettenverteilung:
C12 von 48 - 58 %
C14 von 18 - 24 %
C16 von 8 - 12 %
C18 von 11 -15% und
c) gegebenenfalls 5 Gew. % eines aliphatischen Diols mit mindestens 3 Kohlenstoffatomen,
mit der Maßgabe, dass das Gemisch einen nach dynamischer Differenzkalorimetrie (DSC) gemessenen Schmelzbereich zwischen -20 °C und +70°C aufweist, wobei die Breite des Schmelzbereichs mindestens 30 Temperaturgrade umfasst und das Maximum des Schmelzbereichs bei 35 ± 15 °C liegt,
wobei die Startalkohole (a) bis (c) in einer Guerbet-Reaktion bis zu einem Umsatz von 60 bis 80% umgesetzt werden.

2. Guerbetalkoholgemisch gemäß Anspruch 1, erhältlich durch Umsetzung von
a) 60 bis 70 Gew. % Cetylstearylalkohol,
b) 30 bis 40 Gew.% unverzweigten, gesättigten Fettalkoholen mit folgender Kettenverteilung:
C12 von 48 - 58 %
C14 von 18 - 24 %
C16 von 8 - 12 %
C18 von 11 -15% und
c) gegebenenfalls 5 Gew.% eines aliphatischen Diols mit mindestens 3 Kohlenstoffatomen.

3. Guerbetalkoholgemisch gemäß den Ansprüchen 1 und/oder 2, **dadurch gekennzeichnet, dass** es einen Schmelzbereich zwischen -10 °C und +60 °C aufweist, wobei die Breite des Schmelzbereichs mindestens 40 Temperaturgrade umfasst und das Maximum des Schmelzbereichs bei 35 ± 10 °C liegt.

4. Guerbetalkoholgemisch gemäß den Ansprüchen 1 bis 3, **dadurch gekennzeichnet, dass** die Komponente a) aus unverzweigten Fettalkoholen mit einer Kettenverteilung von
C16 von 45-55 % und
C18 von 45-55 % besteht.

5. Guerbetalkoholgemisch gemäß den Ansprüchen 1 bis 4, **dadurch gekennzeichnet, dass** als Komponente c) Hexandiol eingesetzt wird.

6. Kosmetische und/oder pharmazeutische Zubereitungen enthaltend Guerbetalkoholgemische gemäß einem der Ansprüche 1 bis 5.

7. Verwendung von Guerbetalkoholgemischen gemäß einem der Ansprüche 1 bis 5 als Ersatz von Vaseline in kosmetischen oder pharmazeutischen Zubereitungen.

## Claims

1. A Guerbet alcohol mixture obtainable by reacting
a) 55 to 95% by weight of cetylstearyl alcohol,
b) 5 to 45% by weight of unbranched, saturated fatty alcohols having the following chain distribution:
C12 from 48-58%
C14 from 18-24%
C16 from 8-12%
C18 from 11-15%,
and
c) optionally 5% by weight of an aliphatic diol having at least 3 carbon atoms
with the proviso that the mixture has a melting range, measured by differential scanning calorimetry (DSC), between -20°C and +70°C, where the width of the melting range comprises at least 30 temperature degrees and the maximum of the melting range is 35 ± 15°C,
wherein the starting alcohols (a) to (c) are converted in a Guerbet reaction up to a conversion of 60 to 80%.

2. The Guerbet alcohol mixture according to c claim 1 obtainable by reacting
a) 60 to 70% by weight of cetylstearyl alcohol,
b) 30 to 40% by weight of unbranched, saturated fatty alcohols having the following chain distribution:
C12 from 48-58%
C14 from 18-24%
C16 from 8-12%
C18 from 11-15%,
and
c) optionally 5% by weight of an aliphatic diol having at least 3 carbon atoms.

3. The Guerbet alcohol mixture according to claims 1 and/or 2, wherein it has a melting range between - 10°C and +60°C, where the width of the melting range comprises at least 40 temperature degrees and the maximum of the melting range is 35 ± 10°C.

4. The Guerbet alcohol mixture according to claims 1 to 3, wherein the component a) consists of unbranched fatty alcohols with a chain distribution of
C16 of 45-55% and
C18 of 45-55%.

5. The Guerbet alcohol mixture according to claims 1 to 4, wherein hexanediol is used as component c).

6. A cosmetic and/or pharmaceutical preparation comprising Guerbet alcohol mixtures according to any one of claims 1 to 5.

7. The use of Guerbet alcohol mixtures according to any one of claims 1 to 5 to replace Vaseline in cosmetic or pharmaceutical preparations.

## Revendications

1. Mélange d'alcools de Guerbet, pouvant être obtenu par mise en réaction de
a) 55 à 95 % en poids d'alcool cétylstéarylique,
b) 5 à 45 % en poids d'alcools gras saturés non ramifiés ayant la distribution de chaînes suivante :
C12 de 48 à 58 %
C14 de 18 à 24 %
C16 de 8 à 12 %
C18 de 11 à 15 % et
c) éventuellement 5 % en poids d'un diol aliphatique contenant au moins 3 atomes de carbone,
à condition que le mélange présente une plage de fusion mesurée par calorimétrie différentielle dynamique (DSC) comprise entre -20 °C et +70 °C, la largeur de la plage de fusion comprenant au moins 30 degrés de température et le maximum de la plage de fusion se situant à 35 ± 15 °C,
les alcools de départ (a) à (c) étant mis en réaction dans une réaction de Guerbet jusqu'à une conversion de 60 à 80 %.

2. Mélange d'alcools de Guerbet selon la revendication 1, pouvant être obtenu par mise en réaction de
a) 60 à 70 % en poids d'alcool cétylstéarylique,
b) 30 à 40 % en poids d'alcools gras saturés non ramifiés ayant la distribution de chaînes suivante :
C12 de 48 à 58 %
C14 de 18 à 24 %
C16 de 8 à 12 %
C18 de 11 à 15 % et
c) éventuellement 5 % en poids d'un diol aliphatique contenant au moins 3 atomes de carbone.

3. Mélange d'alcools de Guerbet selon les revendications 1 et/ou 2, **caractérisé en ce qu'**il présente une plage de fusion comprise entre -10 °C et +60 °C, la largeur de la plage de fusion comprenant au moins 40 degrés de température et le maximum de la plage de fusion se situant à 35 ± 10 °C.

4. Mélange d'alcools de Guerbet selon les revendications 1 à 3, **caractérisé en ce que** le composant a) est constitué d'alcools gras non ramifiés ayant la distribution de chaînes suivante :
C16 de 45 à 55 % et
C18 de 45 à 55 %.

5. Mélange d'alcools de Guerbet selon les revendications 1 à 4, **caractérisé en ce que** de l'hexanediol est utilisé en tant que composant c).

6. Préparations cosmétiques et/ou pharmaceutiques contenant des mélanges d'alcools de Guerbet selon l'une quelconque des revendications 1 à 5.

7. Utilisation de mélanges d'alcools de Guerbet selon l'une quelconque des revendications 1 à 5 en tant que substitut de vaseline dans des préparations cosmétiques ou pharmaceutiques.
